(19) Europäisches Patentamt
European Patent Office
Office européen des brevets

(11) Veröffentlichungsnummer: **0 254 950 B1**

(12) **EUROPÄISCHE PATENTSCHRIFT**

(45) Veröffentlichungstag der Patentschrift: **16.10.91**

(51) Int. Cl.[5]: **C07C 69/54**, C07C 271/06, A61K 6/08, C08F 20/28, C07C 67/08, C08G 18/10

(21) Anmeldenummer: **87110144.0**

(22) Anmeldetag: **14.07.87**

# (54) (Meth)-acrylsäureester.

(30) Priorität: **25.07.86 DE 3625203**
**03.02.87 DE 3703080**

(43) Veröffentlichungstag der Anmeldung:
**03.02.88 Patentblatt 88/05**

(45) Bekanntmachung des Hinweises auf die Patenterteilung:
**16.10.91 Patentblatt 91/42**

(84) Benannte Vertragsstaaten:
**AT BE CH DE ES FR GB IT LI NL SE**

(56) Entgegenhaltungen:
**BE-A- 664 445**
**US-A- 4 250 322**

(73) Patentinhaber: **BAYER AG**

**W-5090 Leverkusen 1 Bayerwerk(DE)**

(72) Erfinder: **Reiners, Jürgen, Dr.**
**Carl-Rumpff-Strasse 57**
**W-5090 Leverkusen 1(DE)**
Erfinder: **Podszun, Wolfgang, Dr.**
**Wolfskaul 4**
**W-5000 Koeln 80(DE)**
Erfinder: **Winkel, Jens, Dr.**
**Hahnenweg 6**
**W-5000 Koeln 80(DE)**

**EP 0 254 950 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft die Verwendung spezieller (Meth)-acrylsäureester als monomere Komponenten für Dentalwerkstoffe, sowie die erhaltenen neuen Dentalwerkstoffe selbst.

Die Verwendung von polyfunktionellen (Meth)-acrylsäurederivaten als Komponenten für Zahnfüllungsmaterialien ist bekannt. So werden in der EP-A 00 17 936 Acrylsäureester und Methacrylsäureester vom Pentaerythrit beschrieben. Die dort beschriebenen Monomeren ergeben in Kombination mit anorganischen Füllstoffen Dentalwerkstoffe, die einen unerwünschten Polymerisationsschrumpf aufweisen, der zu einer Spaltbildung zwischen Zahn- und Füllungsmaterial führte.

In der US 45 54 336 werden Urethangruppen enthaltende (Meth)-acrylsäurederivate für Adhesive im Dentalbereich beschrieben, bei denen die Urethangruppen durch einen eine (Meth)-acrylatgruppe enthaltenden Rest substituiert sind. Diese Verbindungen zeigen als Komponenten in Dentalmassen unzureichende Eigenschaften, inbesondere eine für die Praxis zu geringe Festigkeit.

Aus der BE-A 664 445 und der US-P 4,250,322 sind polyfunktionelle Methacrylatgruppen enthaltende Monomere bekannt geworden, die zusätzlich Urethangruppen enthalten. Diese werden zur Herstellung von photopolymerisierbaren Zubereitungen zur Herstellung von Reliefdruckplatten und ähnlichen Reproduktionselementen verwendet.

Es wurde nun gefunden, daß (Meth)-acrylsäureester der Formel

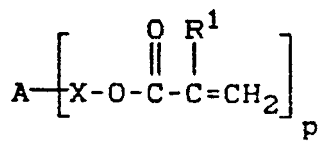

(I),

in der

| | |
|---|---|
| A | ein geradkettiger oder verzweigter, gegebenenfalls 1 oder 2 Sauerstoffbrücken enthaltender, aliphatischer Rest mit 3 bis 20 Kohlenstoffatomen oder ein araliphatischer Rest mit 7 bis 26 Kohlenstoffatomen oder ein cycloaliphatischer Rest mit 6 bis 26 Kohlenstoffatomen ist, |
| p | für die Anzahl der von A ausgehenden Ketten steht und eine Zahl von 3 bis 6 bedeutet, |
| $R^1$ | für jede von A ausgehende Kette unabhängig Wasserstoff oder Methyl bedeutet und |
| X | für einen der Reste |

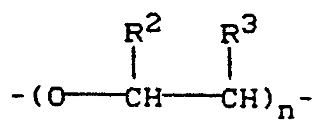

,

oder

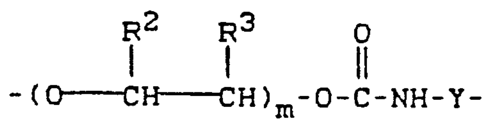

,

steht,
worin

| | |
|---|---|
| n | für jede von A ausgehende Kette unabhängig eine Zahl von 1 bis 5 bedeutet, |
| m | für jede von A ausgehende Kette unabhängig eine Zahl von 0 bis 5 bedeutet, |
| $R^2$ und $R^3$ | Wasserstoff oder Wasserstoff und Methyl bedeuten und |
| Y | ein zweiwertiger, geradkettiger oder verzweigter, aliphatischer Rest mit 2 bis 12 Kohlenstoffatomen, der gegebenenfalls 1 oder 2 Sauerstoffbrücken enthalten kann, ist |

Dentalmaterialien hervorragende Eigenschaften verleihen.

Dentalmassen, bei denen von diesen (Meth)-acrylsäureestern ausgegangen wird, zeigen überraschenderweise einen wesentlich geringeren Polymerisationsschrumpf und größere Festigkeit, und sind daher für die Anwendung in der Praxis besonders geeignet.

Im Rahmen der vorliegenden Erfindung können die Substituenten die folgende Bedeutung haben:

Ein aliphatischer Rest (A) kann ein geradkettiger oder verzweigter Kohlenwasserstoffrest mit 3 bis 20, bevorzugt 3 bis 12, Kohlenstoffatomen bedeuten. Beispielsweise seien die folgenden aliphatischen Reste genannt:

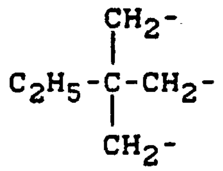 , 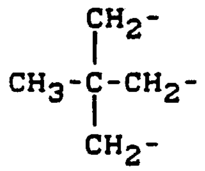 , 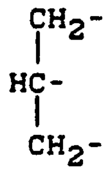 , 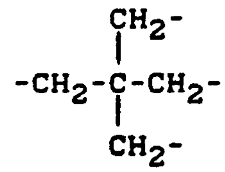 ,

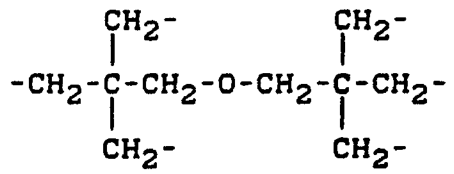 , 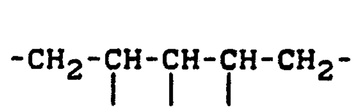 .

Ein araliphatischer Rest (A) kann einen Kohlenwasserstoffrest mit einem geradkettigen oder verzweigten aliphatischen und einem aromatischen Teil mit 7 bis 26 Kohlenstoffatomen bedeuten, wobei der aromatische Teil bevorzugt 6 bis 12 und der aliphatische Teil bevorzugt 1 bis 14 Kohlenstoffatome enthält. Beispielsweise seien die folgenden araliphatischen Reste genannt:

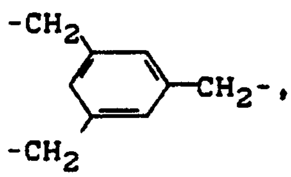 , 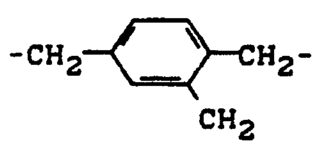 ,

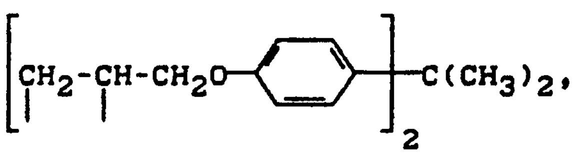 ,

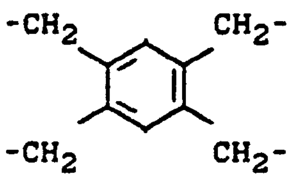 , 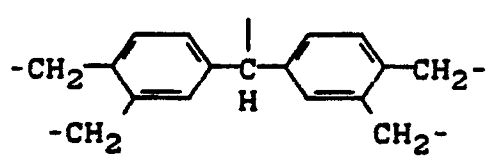

Ein cycloaliphatischer Rest (A) kann einen cyclischen Kohlenwasserstoffrest mit 6 bis 26 Kohlenstoffatomen, bevorzugt 6 bis 12 Kohlenstoffatomen, bedeuten. Beispielsweise seien die folgenden cycloaliphatischen Reste genannt:

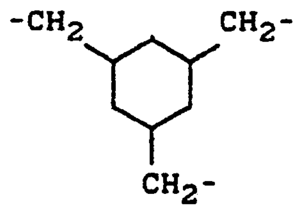 , 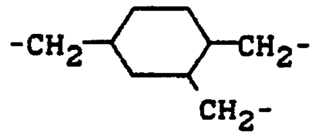

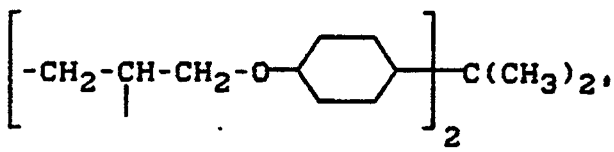

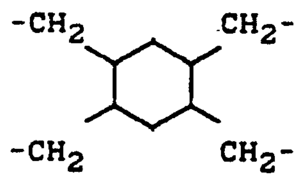 , 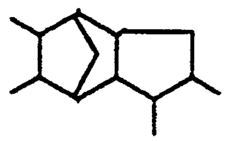 ,

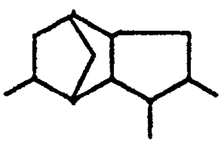 , 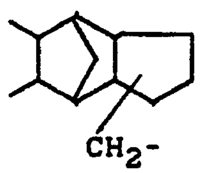 ,

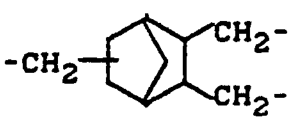 ,

Die Reste A können 1 oder 2 Sauerstoffatome enthalten, so daß aliphatische, cycloaliphatische bzw. araliphatische Ether vorliegen.

Insbesondere bevorzugt seien die folgenden Reste A genannt:

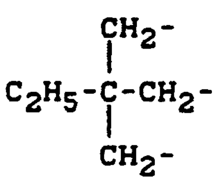 , 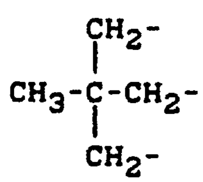 , 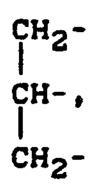 , 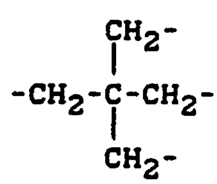 und

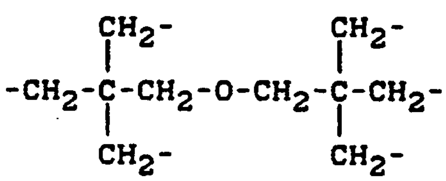 , 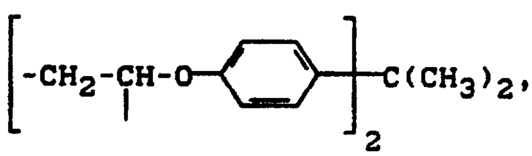

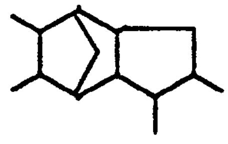 ,

Ein zweiwertiger aliphatischer Rest (Y) kann einen geradkettigen oder verzweigten Kohlenwasserstoffrest mit 2 bis 12 Kohlenstoffatomen, bevorzugt 2 bis 10 Kohlenstoffatomen, bedeuten. Beispielsweise seien die folgenden zweiwertigen aliphatischen Reste genannt: Ethylen, Propylen, 1,4-Tetramethylen, 1,6-Hexamethylen und 1,2-Dimethylpropan-1,3-diyl.

Der Rest Y kann 1 oder 2, bevorzugt 1, Sauerstoffatom enthalten. Beispielsweise seien genannt:

$-CH_2CH_2-O-CH_2CH_2-$, 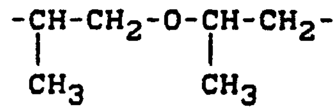 ,

Bevorzugt werden (Meth)-acrylsäureester der Formel (I), bei denen

A ein geradkettiger oder verzweigter, gegebenenfalls 1 oder 2 Sauerstoffbrücken enthaltender, aliphatischer Rest mit 3 bis 12 Kohlenstoffatomen oder ein araliphatischer Rest mit 7 bis 26 Kohlenstoffatomen oder ein cycloaliphatischer Rest mit 6 bis 12 Kohlenstoffatomen ist,

p für die Anzahl der von A ausgehenden Ketten steht und eine Zahl von 3 bis 6 bedeutet,

$R^1$ für jede von A ausgehende Kette unabhängig Wasserstoff oder Methyl bedeutet und

X für einen der Reste

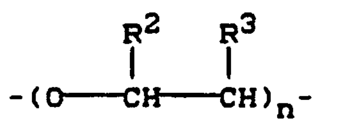 ,

oder

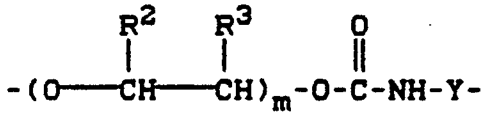 ,

worin

n für jede von A ausgehende Kette unabhängig eine Zahl von 1 bis 5 bedeutet,

m für jede von A ausgehende Kette unabhängig eine Zahl von 0 bis 5 bedeutet,

$R^2$ und $R^3$ Wasserstoff oder Wasserstoff und Methyl bedeuten und

Y ein zweiwertiger, geradkettiger oder verzweigter, aliphatischer Rest mit 2 bis 10 Kohlenstoffatomen, der gegebenenfalls 1 oder 2 Sauerstoffbrücken enthalten kann, ist

steht.

Insbesondere bevorzugt werden (Meth)-acrylsäureester der Formel (I), bei denen

A ein geradkettiger oder verzweigter, gegebenenfalls 1 Sauerstoffbrücke enthaltender aliphatischer Rest mit 3 bis 12 Kohlenstoffatomen ist,

p für die Anzahl der von A ausgehenden Ketten steht und eine Zahl von 3 bis 6 bedeutet,

$R^1$ für jede von A ausgehende Kette unabhängig Wasserstoff oder Methyl bedeutet,

und

X für einen der Reste

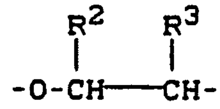

,

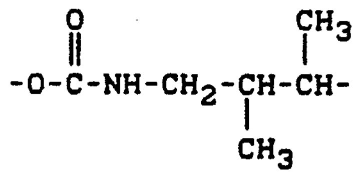

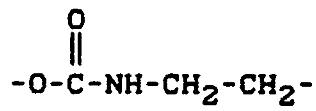

oder

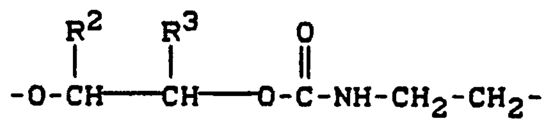

worin

$R^2$ und $R^3$ Wasserstoff oder Wasserstoff und Methyl bedeuten,

steht.

Beispielsweise seien die folgenden (Meth)-acrylsäureester genannt:

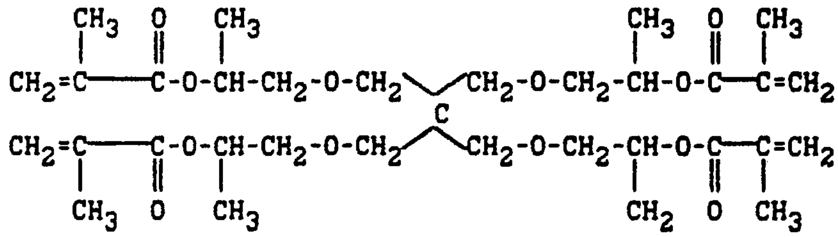

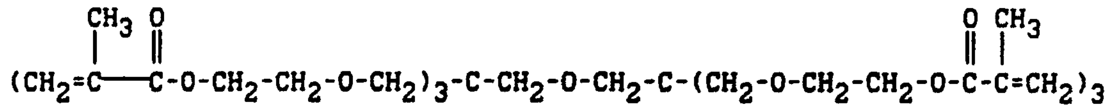

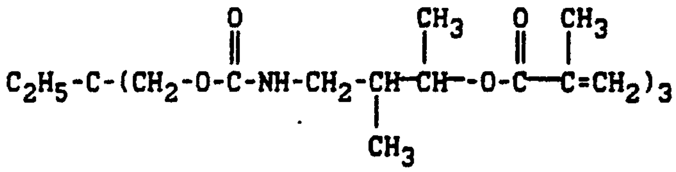

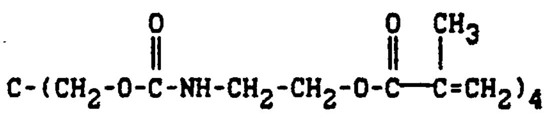

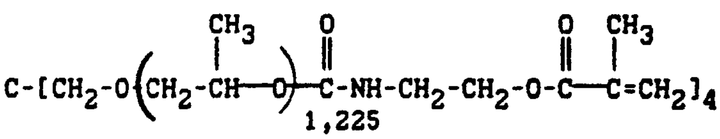

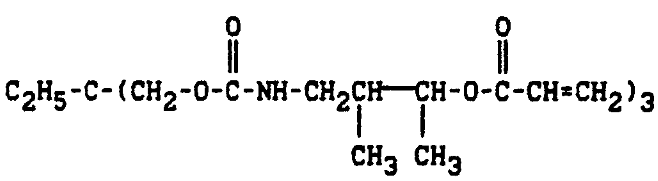

Die erfindungsgemäß verwendeten (Meth)-acrylsäureester der Formel

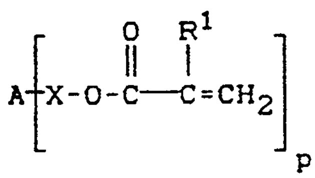

(Ia)

in der

- A ein geradkettiger oder verzweigter, gegebenenfalls 1 oder 2 Sauerstoffbrücken enthaltender, aliphatischer Rest mit 3 bis 20 Kohlenstoffatomen oder ein araliphatischer Rest mit 7 bis 26 Kohlenstoffatomen oder ein cycloaliphatischer Rest mit 6 bis 26 Kohlenstoffatomen ist,
- p für die Anzahl der von A ausgehenden Ketten steht und eine Zahl von 3 bis 6 bedeutet,
- $R^1$ für jede von A ausgehende Kette unabhängig Wasserstoff oder Methyl bedeutet und
- X für den Rest

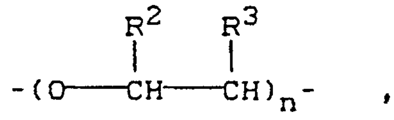

worin

n für jede von A ausgehende Kette unabhängig eine Zahl von 1 bis 5 bedeutet,

$R^2$ und $R^3$ Wasserstoff oder Wasserstoff und Methyl bedeuten,

steht,
werden hergestellt, indem man Polyole der Formel

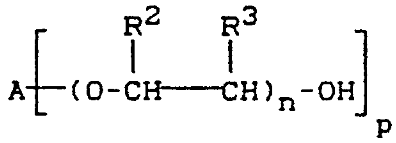

(II),

in der
A, $R^2$, $R^3$, n und p die obengenannte Bedeutung haben,
mit (Meth)-acrylsäure oder (Meth)-acrylsäurederivaten der Formel

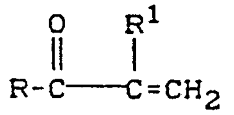

(III),

in der

$R^1$ die obengenannte Bedeutung hat und

R Hydroxy, Halogen, Niederalkoxy oder den Rest

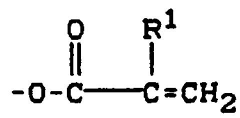

in dem

$R^1$ die obengenannte Bedeutung hat, bedeutet,

in Gegenwart eines Katalysators, eines Polymerisationsinhibitors und eines Lösungsmittels im Temperaturbereich von 50 bis 120° C umsetzt.

Polyole II sind an sich bekannt und können beispielsweise durch Oxyalkylierung der bekannten Polyole $A(OH)_r$ hergestellt werden, wobei die Polyole II auch als Produkte mit variablem Oxyalkylierungsgrad vorliegen können.

Zur Veresterung können (Meth)-acrylsäure, (Meth)-acrylsäurehalogenide, bevorzugt (Meth)-acrylsäurechlorid, (Meth)-acrylsäureanhydrid oder (Meth)-acrylsäureester niederer Alkohole ($C_1$ bis etwa $C_6$), beispielsweise (Meth)-acrylsäuremethylester und (Meth)-acrylsäureethylester, eingesetzt werden.

Katalysatoren sind im Falle der (Meth)Acrylsäure im allgemeinen starke Säuren mit einem $pK_a$-Wert kleiner 2. Beispielsweise seien p-Toluolsulfonsäure, Jodwasserstoff, Schwefelsäure oder stark saure Ionenaustauscher (z.B. mit 2 bis 20 Gew.-% Divinylbenzol vernetzte Polystyrolsulfonsäure) genannt.

Polymerisationsinhibitoren des erfindungsgemäßen Verfahrens sind an sich bekannt. Beispielsweise sei 2,6-Di-tert.-butyl-4-methyl-phenol, Methylenblau und Hydrochinon genannt.

Der saure Katalysator wird im allgemeinen in einer Menge von 0,01 bis 5 Gew.-Teilen bezogen auf 100 Gew.-Teile der Reaktanden eingesetzt.

Der Polymerisationsinhibitor wird im allgemeinen in einer Menge von 0,01 bis 1, bevorzugt 0,01 bis 0,2 Gew.-Teilen, bezogen auf 100 Gew.-Teile der Reaktanden eingesetzt.

In diesem Verfahren anwendbare Lösungsmittel sind im allgemeinen solche, die mit Wasser bei Raumtemperatur nicht mischbar sind. Beispielsweise sei Toluol, Chloroform, Xylol, Chlorbenzol, Methylethylketon genannt.

Dieses Verfahren wird im allgemeinen im Temperaturbereich von 50 bis 120 ° C, bevorzugt von 70 bis 110 ° C, durchgeführt. Im allgemeinen wird das erfindungsgemäße Verfahren bei Normaldruck durchgeführt.

Dieses Verfahren wird im allgemeinen mit einem Überschuß an (Meth)-acrylsäure durchgeführt. Im allgemeinen setzt man 1 bis 2 Mol (Meth)-acrylsäure bezogen auf jede Hydroxylgruppe pro Mol des Polyols II ein.

Das Verfahren kann beispielsweise wie folgt durchgeführt werden:
Das Polyol und die (Meth)-acrylsäure werden in einem Lösungsmittel suspendiert oder gelöst und mit dem sauren Katalysator und dem Polymerisationsinhibitor versetzt. Das während der Veresterung gebildete Wasser kann durch azeotrope Destillation aus dem Gleichgewicht entfernt werden.

Nach beendeter Veresterung wird nicht umgesetzte (Meth)-acrylsäure durch Extraktion mit einer basischen wäßrigen Lösung (z.B. Sodalösung) entfernt. Der Inhibitor wird beispielsweise durch Zusatz von Adsorbenzien (z.B. Bleicherde, Celite, Silicagel, Aluminiumoxid) abgetrennt.

Die Aufarbeitung der (Meth)-acrylsäureester erfolgt in an sich bekannter Weise durch Abdestillieren der Lösungsmittel.

Im Falle der (Meth)Acrylsäurederivate mit R der Bedeutung Niederalkoxy sind die obengenannten Katalysatoren oder Titanalkoholate wie Tetrabutoxy-titan geeignet.

Der bei der Reaktion gebildete Alkohol, der sich vom Niederalkoxyrest des (Meth)acrylsäurederivats (III) ableitet, wird azeotrop abdestilliert. Nach beendeter Reaktion wird das nicht umgesetzte (Meth)Acrylsäure-Derivat im Vakuum abdestilliert und der Rückstand in an sich bekannter Weise aufgearbeitet.

Im Falle der (Meth)Acrylsäure-Derivate mit R in der Bedeutung Halogen bzw.

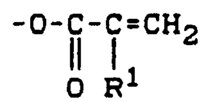

also z.B. bei der Veresterung mit (Meth)Acrylsäurechlorid bzw. (Meth)Acrylsäureanhydrid wird im Gegensatz zu den beiden zuvor genannten Verfahrensvarianten bevorzugt bei niedrigeren Temperaturen mit dem Polyol verestert. Bei Verwendung von (Meth)Acrylsäurechlorid wird bevorzugt ein tertiäres Amin, z.B. Triethylamin, Dicyclohexylmethylamin usw., als Säureakzeptor in stöchiometrischen Mengen eingesetzt. Bei Verwendung von (Meth)Acrylsäureanhydrid werden gegebenenfalls weniger als 10 % des tertiären Amins eingesetzt. Das Produkt wird in bekannter Weise durch Extraktion des Reaktionsgemisches mit einer wäßrigen basischen Lösung gereinigt und wie in der ersten Verfahrensvariante beschrieben, aufgearbeitet.

Ein weiteres Verfahren zur Herstellung von (Meth)-acrylsäureester der Formel

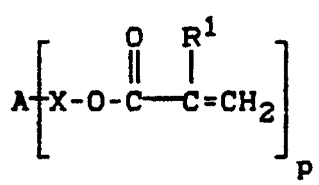

(Ib)

in der

A ein geradkettiger oder verzweigter, gegebenenfalls 1 oder 2 Sauerstoffbrücken enthaltender, aliphatischer Rest mit 3 bis 20 Kohlenstoffatomen oder ein araliphatischer Rest mit 7 bis 26 Kohlenstoffatomen oder ein cycloaliphatischer Rest mit 6 bis 26 Kohlenstoffatomen ist,

p für die Anzahl der von A ausgehenden Ketten steht und eine Zahl von 3 bis 6 bedeutet,

$R^1$ für jede von A ausgehende Kette unabhängig Wasserstoff oder Methyl bedeutet und

X für den Rest

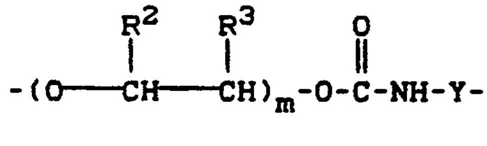

worin

m für jede von A ausgehende Kette unabhängig eine Zahl von 0 bis 5 bedeutet,

$R^2$ und $R^3$ Wasserstoff oder Wasserstoff und Methyl bedeutet,

Y ein zweiwertiger, geradkettiger oder verzweigter, aliphatischer Rest mit 2 bis 12 Kohlenstoffatomen, der gegebenenfalls 1 oder 2 Sauerstoffbrücken enthalten kann, ist

steht,

ist dadurch gekennzeichnet, daß man Polyole der Formel

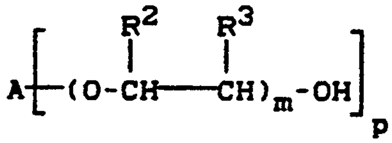 (II),

in der

A, $R^2$, $R^3$, m und p die obengenannte Bedeutung haben,

mit Isocyanatgruppen enthaltendem (Meth)-acrylsäureester der Formel

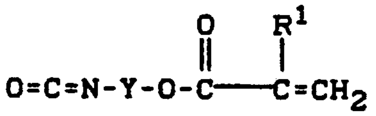 (IV)

in der

$R^1$ und Y die obengenannte Bedeutung haben,

in Gegenwart eines Katalysators, eines Polymerisationsinhibitors und eines Lösungsmittels im Temperaturbereich von 20 bis 100° C umsetzt.

Polyole für das zuletzt genannte Verfahren sind an sich bekannt und können beispielsweise durch Oxyalkylierung der entsprechenden Polyole hergestellt werden.

Isocyanatgruppen enthaltende (Meth)-acrylsäureester der Formel (IV) sind an sich bekannt (DE-A 333 8077) und können beispielsweise durch Phosgenierung der zugrundeliegenden 5,6-Dihydro-oxazine hergestellt werden.

Katalysatoren können hier zinnhaltige Katalysatoren, wie Dibutylzinndilaurat oder Zinn(II)-octoat sein. Es ist auch möglich als Katalysatoren Verbindungen mit tert.-Aminogruppen, beispielsweise Triethylamin, Triethylendiamin, und Titanverbindungen, wie Titantetraisopropylat oder Titantetraethanolat einzusetzen.

Im allgemeinen wird der Katalysator in einer Menge von 0,01 bis 2,5 Gew.-%, bevorzugt von 0,1 bis 1,5 Gew.-%, bezogen auf die Gesamtmenge der Reaktanden eingesetzt.

Als Polymerisationsinhibitoren seien beispielsweise 2,6-Di-tert.-butyl-4-methyl-phenol, Methylenblau und Hydrochinon genannt. Der Polymerisationsinhibitor wird im allgemeinen in einer Menge von 0,01 bis 1,0 Gew.-%, bevorzugt von 0,01 bis 0,2 Gew.-%, bezogen auf die Gesamtmenge der Reaktanden eingesetzt.

Im allgemeinen setzt man den Isocyanatgruppen enthaltenden (Meth)-acrylsäureester IV in einer Menge von P Mol, bezogen auf 1 Mol des Polyols ein, so daß eine stöchiometrische Äquivalenz von NCO- und OH-Gruppen besteht.

Das zuletzt genannte Verfahren führt man vorzugsweise unter Wasserausschluß in einem inerten Lösungsmittel durch. Als inerte Lösungsmittel seien beispielsweise Chloroform, Tetrahydrofuran, Dioxan, Methylenchlorid, Toluol und Acetonitril genannt. Bevorzugte Lösungsmittel sind Chloroform, Toluol und Acetonitril.

Das zuletzt genannte Verfahren kann im allgemeinen im Temperaturbereich von 20 bis 100° C, bevorzugt von 30 bis 70° C, durchgeführt werden.

Das zuletzt genannte Verfahren wird im allgemeinen bei Normaldruck durchgeführt.

Das zuletzt genannte Verfahren kann beispielsweise wie folgt durchgeführt werden:
Das Polyol und der entsprechende Isocyanatgruppen enthaltende (Meth)-acrylsäureester werden in einem Lösungsmittel gelöst oder suspendiert und unter Rühren mit dem Katalysator versetzt.

Nach Zugabe des Polymerisationsinhibitors wird die Reaktionslösung auf eine erfindungsgemäße Temperatur erwärmt.

Der zeitliche Verlauf der Umsetzung kann beispielsweise durch Messung der IR-Spektren verfolgt werden. Nach vollständiger Umsetzung der Isocyanatgruppen werden die Reaktionsprodukte durch Entfernen des Lösungsmittels in Vakuum isoliert.

Die Urethan-(Meth)-acrylsäureester können insbesondere als Monomere für Dentalwerkstoffe verwendet werden. Als Dentalwerkstoffe seien beispielsweise Füllungsmaterialien für Zähne, Beschichtungsmittel für Zähne und Komponenten für die Herstellung von Zahnersatz, bevorzugt Kunststoffzähne, genannt. Je nach Anwendungsgebiet können Dentalwerkstoffe weitere Additive enthalten.

Für die Anwendung als Monomere für polymerisierbare Zahlfüllmassen oder Beschichtungsmittel im Dentalbereich können die erfindungsgemäßen polyfunktionellen (Meth)-acrylsäureester mit an sich bekannten Monomeren gemischt werden, um beispielsweise die Viskosität dem Verwendungszweck anzupassen. Hierbei werden Viskositäten im Bereich von 60 bis 10.000 mPas bevorzugt. Dieses ist dadurch erreichbar, daß man den erfindungsgemäßen Monomeren gegebenenfalls ein Comonomer niedriger Viskosität als Reaktivverdünner bzw. Lösungsmittel zumischt. Die erfindungsgemäßen Verbindungen werden in der Mischung mit Comonomeren mit einem Anteil von ca. 30 bis ca. 90 Gew.-%, bevorzugt von 40 bis 80 Gew.-% eingesetzt. Zweck der vorliegenden Erfindung ist es, ebenfalls bevorzugt Mischungen verschiedener erfindungsgemäßer (Meth)-acrylsäurederivate einzusetzen.

Es ist auch möglich, Monomermischungen einzusetzen, die mehrere Comonomere enthalten, um die gewünschte Viskosität zu erreichen.

Beispielsweise seien die folgenden Comonomeren genannt: Glycerindimethacrylat, Triethylenglykoldimethacrylat, Tetraethylenglykoldimethylacrylat, 1,12-Dodecandioldimethacrylat, 1,6-Hexandioldimethacrylat, Diethylenglykoldimethacrylat, 2,2-Bis-[p-(2'-hydroxy-3'-methacryloyloxypropoxy)-phenyl]-propan, 2,2-Bis-[p-(2'-methacryloyloxyethoxy)-phenyl]-propan, Trimethylol-propan-tri-(meth)-acrylat, Bis-(meth)-acryloyloxyethoxymethyl-tricyclo[5,2,1,$0^{2,6}$]-decan (DE-A 29 31 925 und DE-A 29 31 926).

Darüber hinaus können andere Urethan(meth)acrylsäureester, wie z. B. die 2:1-Addukte aus den genannten Hydroxyalkyl(meth)acrylaten und Diisocyanaten mit 6 bis 14 C-Atomen als Comonomere zugesetzt werden. Als Diisocyanate eignen sich beispielsweise Hexamethylendiisocyanat, Trimethylhexamethylendiisocyanat, Bis-isocyanatomethylcyclohexan, Bis-isocyanatomethyl-tricyclo(5.2.1.$0^{2,6}$)decan.

Insbesondere bevorzugt werden Comonomere, die bei 13 mbar einen Siedepunkt über 100 °C besitzen.

Die erfindungsgemäß verwendeten polyfunktionellen (Meth)-acrylsäureester können gegebenenfalls in Mischung mit den genannten Comonomeren mit an sich bekannten Methoden zu vernetzten Polymerisaten aushärten (Am. Chem. Soc., Symp. Ser. 212, 359-371 (1983)). Für die sogenannte Redoxpolymerisation ist ein System aus einer peroxidischen Verbindung und einem Reduktionsmittel, beispielsweise auf Basis tertiärer aromatischer Amine, geeignet. Beispiele für Peroxide sind: Dibenzoylperoxid, Dilauroylperoxid und Di-4-chlorbenzoylperoxid.

Als tertiäre aromatische Amine seien beispielsweise N,N-Dimethyl-p-toluidin, Bis-(2-hydroxyethyl)-p-toluidin, Bis-(2-hydroxyethyl)-3,5-dimethylanilin und N-Methyl-N-(2-methylcarbamoyloxypropyl)-3,5-dimethylanilin genannt. Die Konzentrationen des Peroxids bzw. des Amins werden vorteilhaft so gewählt, daß sie 0,1 bis 5 Gew.-%, bevorzugt 0,5 bis 3 Gew.-%, bezogen auf die Monomermischung betragen.

Die peroxid- bzw. aminhaltigen Monomermischungen werden bis zur Anwendung getrennt gelagert.

Die erfindungsgemäß verwendeten Polymeren können auch durch Bestrahlung mit UV-Licht oder sichtbarem Licht (beispielsweise im Wellenlängenbereich von 230 bis 650 nm) zur Polymerisation gebracht werden. Als Initiatoren für die photoinitiierte Polymerisation eignen sich beispielsweise Benzil, Benzildimethylketal, Benzoinmonoalkylether, Benzophenon, p-Methoxybenzophenon, Fluorenon, Thioxanthon, Phenanthrenchinon und 2,3-Bornandion (Campherchinon), gegebenenfalls in Gegenwart von synergistisch wirkenden Aktivatoren, wie N,N-Dimethylaminoethylmethacrylat, Triethanolamin, 4-N,N-Dimethylaminobenzolsulfonsäurediallylamid. Die Durchführung der Photopolymerisation ist beispielsweise in der DE-A 3 135 115 beschrieben.

Neben den oben beschriebenen Initiatoren können den erfindungsgemäßen (Meth)-acrylsäure-Derivaten an sich für diesen Einsatzzweck bekannte Lichtschutzmittel und Stabilisatoren zugesetzt werden.

Lichtschutzmittel sind beispielsweise in (Gächter, Müller, Taschenbuch der Kunststoff-Additive, 2. Ausgabe, Carl Hanser Verlag) beschrieben. Beispielsweise seien die folgenden Lichtschutzmittel genannt: Cyasorb UV9®, Tinuvin P®, Tinuvin 770®, Tinuvin 622®, Tinuvin 765®.

Stabilisatoren sind beispielsweise in (Ullmanns Encyclopädie der technischen Chemie, 4, Auflage, Bd.

8) beschrieben. Beispielsweise seien die folgenden Stabilisatoren genannt: 2,6-Di-tert.-butylphenol, 2,6-Ditert.-butyl-4-methylphenol,2,6-Di-octadecyl-4-methyl-phenol, 1,1'-Methylen-bis(naphthol-2) u.a.

Die Lichtschutzmittel und die Stabilisatoren können jeweils in einer Menge von 0,01 bis 0,5 Gew.-Teilen, bezogen auf 100 Gew.-Teile der Monomermischung, eingesetzt werden.

Die Monomermischungen können ohne Zusatz von Füllstoffen als Beschichtungsmittel (Zahnlacke) eingesetzt werden.

Bei der Verwendung als Zahnfüllmassen setzt man den erhaltenen Monomermischungen im allgemeinen Füllstoffe zu. Um einen hohen Füllgrad erreichen zu können, sind Monomermischungen, die eine Viskosität im Bereich von 60 bis 10.000 mPas besitzen, besonders vorteilhaft.

Vorzugsweise mischt man den (Meth)acrylsäurederivaten anorganische Füllstoffe zu. Beispielsweise seien Bergkristall, Kristoballit, Quarzglas, hochdisperse Kieselsäure, Aluminiumoxid und Glaskeramiken, beispielsweise Zirkon enthaltende Glaskeramiken (DE-A 23 47 591) genannt. Die anorganischen Füllstoffe werden zur Verbesserung des Verbunds zur Polymermatrix des Polymethacrylats, vorzugsweise mit einem Haftvermittler vorbehandelt. Die Haftvermittlung kann beispielsweise durch eine Behandlung mit Organosiliciumverbindungen erreicht werden (Progress in Organic Coatings 11, 297-308 (1983)). Bevorzugt wird 3-Methacryloyloxypropyl-trimethoxysilan eingesetzt. Die Füllstoffe für die erfindungsgemäßen Zahnfüllmassen weisen im allgemeinen einen mittleren Teilchendurchmesser von 0,01 bis 100 µm, vorzugsweise von 0,03 bis 50 µm, besonders bevorzugt von 0,03 bis 5 µm auf. Es kann auch vorteilhaft sein, mehrere Füllstoffe nebeneinander einzusetzen, die einen voneinander verschiedenen mittleren Teilchendurchmesser und/oder einen unterschiedlichen Silangehalt besitzen.

Der Füllstoffanteil in der Zahnfüllmasse beträgt im allgemeinen 5 bis 85 Gew.-%, vorzugsweise 50 bis 80 Gew.-%.

Für die Herstellung der Zahnfüllmassen werden die Komponenten unter Verwendung handelsüblicher Knetmaschinen vermischt.

Der Anteil der Urethan(meth)acrylate in den erfindungsgemäßen Zahnfüllmassen beträgt im allgemeinen 5 bis 70 Gew.-% bezogen auf die Füllmasse.

Die Urethan-(Meth)-Acrylsäure-Derivate können erfindungsgemäß auch als Komponenten bei der Herstellung von Zahnersatz eingesetzt werden.

Dabei werden die erfindungsgemäßen Monomeren mit den üblicherweise verwendeten, an sich bekannten Bestandteilen kombiniert. Vorzugsweise werden die Monomeren im Gemisch mit Alkylmethacrylaten, wie Methylmethacrylat eingesetzt. Es können auch zusätzlich an sich bekannte Perlpolymerisate zugesetzt werden. Zur Einstellung der Zahnfarbe können bekannte anorganische und organische Farbpigmente und Trübungsmittel zugesetzt werden. Auch die Anwendung von Stabilisatoren und Lichtschutzmitteln ist möglich.

Die Kunststoffzähne werden durch radikalische Polymerisation der Dentalmassen unter Formgebung hergestellt.

Die Verarbeitung ist sowohl nach Injektionsverfahren als auch Prägeverfahren möglich und erfolgt im allgemeinen nach den üblichen Herstellungsmethoden für Zähne auf Basis von Poly(methylmethacrylat), z.B. durch thermische Polymerisation unter Verwendung von an sich bekannten Polymerisationsinitiatoren, beispielsweise auf Basis von Peroxiden und Azoverbindungen, wie Dibenzoylperoxid, Dilauroylperoxid, Cyclohexylpercarbonat, Azobisisobuttersäurenitril. Gut geeignet sind auch Mischungen von Polymerisationsinitiatoren mit unterschiedlichen Zerfallstemperaturen.

Die aus den (Meth)Acrylsäureestern erfindungsgemäß hergestellten Dentalwerkstoffe zeichnen sich durch hohe Widerstandsfähigkeit gegenüber mechanischer Beanspruchung und eine hohe Abrasionsbeständigkeit aus.

Beispiel 1

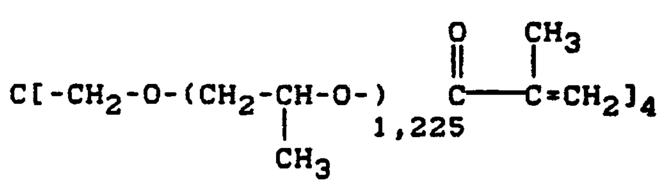

91,5 g des Umsetzungsproduktes aus 1 Mol Pentaerythrit und 4,9 Molen Propylenoxid (OH-Zahl = 534

mg KOH/g) werden in 300 ml Toluol gelöst und mit 1,5 g p-Toluolsulfonsäure, 0,45 g Methylenblau, 0,064 g 2,6-di-tert.-butyl-4-methylphenol und 103,2 g Methacrylsäure versetzt. Das Gemisch wird mit Luft begast und 40 h bei 110-120 ° C gehalten, dabei wird mit Hilfe eines Wasserabscheiders Wasser ausgekreist. Dann wird mit Bleicherde verrührt und abgesaugt. Zur Entfernung überschüssiger Methacrylsäure wird das Filtrat dreimal mit gesättigter Natriumhydrogencarbonat-Lösung extrahiert und mit Wasser neutral gewaschen. Nach trocknung über $Na_2SO_4$ wird mit Aktivkohle behandelt. Der Methacrylsäureester wird als farblose, niedrig-viskose Flüssigkeit isoliert.

| Elementaranalyse | Molgew. |
|---|---|
| gef.: C 61,4 ; H 8,3 | gef.: 640 |
| ber.: C 61,88; H 8,29 | ber.: 692 |

Beispiel 2

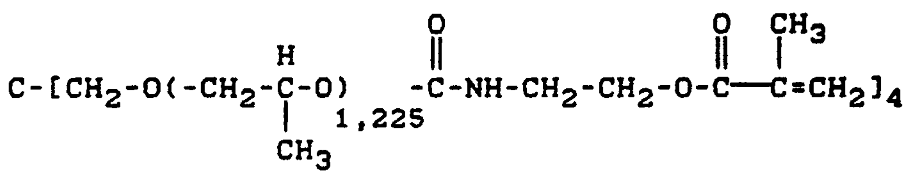

18,3 g des Umsetzungsproduktes aus 1 Mol Pentaerythrit und 4,9 Molen Propylenoxid wird in 50 ml getrocknetem Chloroform gelöst. Nach Zugabe von 0,02 g Jonol und 0,1 g Dibutylzinndilaurat werden langsam 27 g 2-Isocyanatoethylmethacrylat zugetropft. Das Gemisch wird solange bei 50 ° C gerührt, bis kein NCO mehr nachweisbar ist (ca. 150 Stunden). Das Reaktionsprodukt wird über Aktivkohle filtriert und durch Abdampfen des Lösungsmittels isoliert. Man erhält eine viskose Flüssigkeit.

Elementaranalyse
gef.: C 54,5 H 7,3 N 5,5
ber.: C 55,08 % H 7,44 % N 5,38
OH-Zahl: 2 mg KOH/g

Beispiel 3

Herstellung von Kunststoffzähnen

60 Gew.-Teile einer Monomermischung, die aus 45 Gew.-% Triethylenglykoldimethacrylat und aus 55 Gew.-% des Urethan-Methacrylsäure-Derivates aus Beispiel 2 hergestellt wurde, werden mit 1 Gew.-Teil Dibenzoylperoxid und 40 Gew.-Teilen einer mit 5 % 3-Methacryloyloxypropyltrimethoxysilan silanisierten hochdispersen Kieselsäure (BET-Oberfläche: 50 $m^2$/g) vermischt.

Das aktivierte Gemisch wird in eine Zahnform eingespritzt und bei 130 ° C in 6 Minuten ausgehärtet. Die erhaltenen Kunststoffzähne zeigen eine besonders hohe Abrasionsfestigkeit.

**Patentansprüche**
**Patentansprüche für folgende Vertragsstaaten : AT, BE, CH, DE, FR, GB, IT, LI, NL, SE**

1. Verwendung von (Meth)-acrylsäureestern der Formel (I)

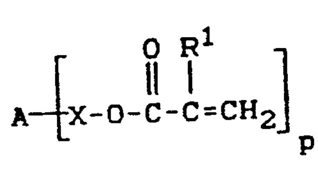
(I)

in der

A ein geradkettiger oder verzweigter, gegebenenfalls 1 oder 2 Sauerstoffbrücken enthaltender, aliphatischer Rest mit 3 bis 20 Kohlenstoffatomen oder ein araliphatischer Rest mit 7 bis 26 Kohlenstoffatomen oder ein cycloaliphatischer Rest mit 6 bis 26 Kohlenstoffatomen ist,

p für die Anzahl der von A ausgehenden Ketten steht und eine Zahl von 3 bis 6 bedeutet,

$R^1$ für jede von A ausgehende Kette unabhängig Wasserstoff oder Methyl bedeutet und

X für einen der Reste

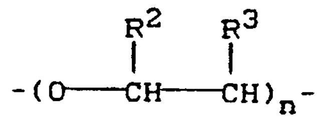 oder

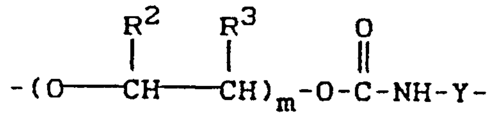 ,

worin

n für jede von A ausgehende Kette unabhängig eine Zahl von 1 bis 5 bedeutet,

m für jede von A ausgehende Kette unabhängig eine Zahl von 0 bis 5 bedeutet und

$R^2$ und $R^3$ Wasserstoff oder Wasserstoff und Methyl bedeuten,

Y ein zweiwertiger, geradkettiger oder verzweigter, aliphatischer Rest mit 2 bis 12 Kohlenstoffatomen, der gegebenenfalls 1 oder 2 Sauerstoffbrücken enthalten kann, ist

steht,

in Dentalwerkstoffen.

2. Verwendung nach Anspruch 1, dadurch gekennzeichnet, daß die (Meth)-acrylsäureester in Zahnfüllmassen eingesetzt werden.

3. Verwendung nach Anspruch 1, dadurch gekennzeichnet, daß die (Meth)-acrylsäureester in Beschichtungsmitteln für Zähne eingesetzt werden.

4. Verwendung nach Anspruch 1, dadurch gekennzeichnet, daß die (Meth)Acrylsäureester zur Herstellung von Kunststoffzähnen verwendet werden.

5. Verwendung von (Meth)-acrylsäureestern der Formel (I)

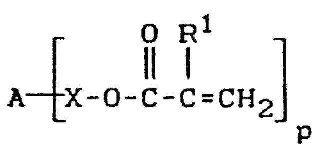 (I)

in der

A ein geradkettiger oder verzweigter, gegebenenfalls 1 oder 2 Sauerstoffbrücken enthaltender, aliphatischer Rest mit 3 bis 20 Kohlenstoffatomen oder ein araliphatischer Rest mit 7 bis 26 Kohlenstoffatomen oder ein cycloaliphatischer Rest mit 6 bis 26 Kohlenstoffatomen ist,

p für die Anzahl der von A ausgehenden Ketten steht und eine Zahl von 3 bis 6 bedeutet,

$R^1$ für jede von A ausgehende Kette unabhängig Wasserstoff oder Methyl bedeutet und

X für einen der Reste

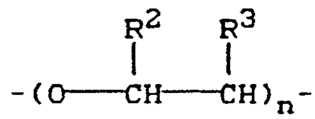 oder

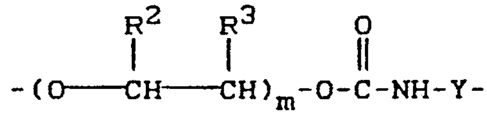 ,

worin

n für jede von A ausgehende Kette unabhängig eine Zahl von 1 bis 5 bedeutet,

m für jede von A ausgehende Kette unabhängig eine Zahl von 0 bis 5 bedeutet,

$R^2$ und $R^3$ Wasserstoff oder Wasserstoff und Methyl bedeuten und

Y ein zweiwertiger, geradkettiger oder verzweigter, aliphatischer Rest mit 2 bis 12 Kohlenstoffatomen, der gegebenenfalls 1 oder 2 Sauerstoffbrücken enthalten kann, bedeutet

steht,

zur Herstellung von Dentalwerkstoffen.

6. Dentalwerkstoffe, dadurch gekennzeichnet, daß sie (Meth)-acrylsäureester der Formel (I)

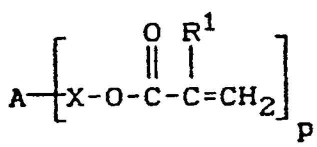 (I)

in der

A ein geradkettiger oder verzweigter, gegebenenfalls 1 oder 2 Sauerstoffbrücken enthaltender, aliphatischer Rest mit 3 bis 20 Kohlenstoffatomen oder ein araliphatischer Rest mit 7 bis 26 Kohlenstoffatomen oder ein cycloaliphatischer Rest mit 6 bis 26 Kohlenstoffatomen ist,

p für die Anzahl der von A ausgehenden Ketten steht und eine Zahl von 3 bis 6 bedeutet,

$R^1$ für jede von A ausgehende Kette unabhängig Wasserstoff oder Methyl bedeutet und

X für einen der Reste

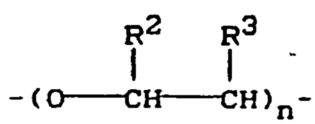 oder

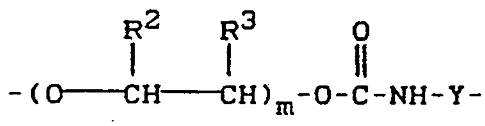 ,

worin

n für jede von A ausgehende Kette unabhängig eine Zahl von 1 bis 5 bedeutet,
m für jede von A ausgehende Kette unabhängig eine Zahl von 0 bis 5 bedeutet,
$R^2$ und $R^3$ Wasserstoff oder Wasserstoff und Methyl bedeuten und
Y ein zweiwertiger, geradkettiger oder verzweigter, aliphatischer Rest mit 2 bis 12 Kohlenstoffatomen, der gegebenenfalls 1 oder 2 Sauerstoffbrücken enthalten kann, bedeutet

steht, enthalten.

**7.** Dentalwerkstoffe nach Anspruch 6, dadurch gekennzeichnet, daß sie neben (Meth)-acrylsäureestern der Formel (I) weitere Comonomere enthalten.

**8.** Dentalwerkstoffe nach den Ansprüchen 6 und 7, dadurch gekennzeichnet, daß sie (Meth)-acrylsäureester der Formel (I), Comonomere und an sich bekannte Additive und gegebenenfalls Füllstoffe enthalten.

**Patentansprüche für folgende Vertragsstaat : ES**

**1.** Verfahren zur Herstellung von Dentalwerkstoffen, dadurch gekennzeichnet, daß

(Meth)-acrylsäureester der Formel (I)

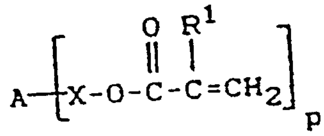 (I)

in der

A ein geradkettiger oder verzweigter, gegebenenfalls 1 oder 2 Sauerstoffbrücken enthaltender, aliphatischer Rest mit 3 bis 20 Kohlenstoffatomen oder ein araliphatischer Rest mit 7 bis 26 Kohlenstoffatomen oder ein cycloaliphatischer Rest mit 6 bis 26 Kohlenstoffatomen ist,
p für die Anzahl der von A ausgehenden Ketten steht und eine Zahl von 3 bis 6 bedeutet,
$R^1$ für jede von A ausgehende Kette unabhängig Wasserstoff oder Methyl bedeutet und
X für einen der Reste

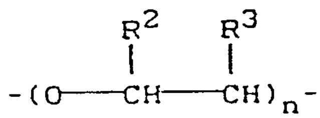 oder

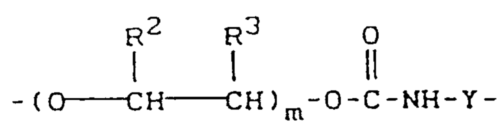 ,

worin

n für jede von A ausgehende Kette unabhängig eine Zahl von 1 bis 5 bedeutet,
m für jede von A ausgehende Kette unabhängig eine Zahl von 0 bis 5 bedeutet und
$R^2$ und $R^3$ Wasserstoff oder Wasserstoff und Methyl bedeuten,
Y ein zweiwertiger, geradkettiger oder verzweigter, aliphatischer Rest mit 2 bis 12 Kohlenstoffatomen, der gegebenenfalls 1 oder 2 Sauerstoffbrücken enthalten kann, ist

steht, gegebenenfalls in Abmischung mit Comonomeren, an sich bekannten Additiven und Füllstoffen, auf an sich bekannte Weise ausgehärtet werden.

2. Verfahren gemäß Anspruch 1, dadurch gekennzeichnet, daß es sich bei dem Dentalwerkstoff um einen künstlichen Zahn handelt.

**Claims**
**Claims for the following Contracting States : AT, BE, CH, DE, FR, GB, IT, LI, NL, SE**

1. Use of (meth)acrylic acid esters of the formula

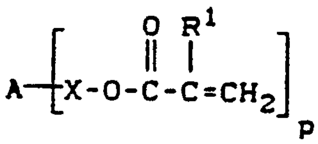

(I)

in which

- A is a straight-chain or branched aliphatic radical having 3 to 20 carbon atoms and optionally containing 1 or 2 oxygen bridges, an araliphatic radical having 7 to 26 carbon atoms or a cycloaliphatic radical having 6 to 26 carbon atoms,
- p represents the number of chains starting from A and denotes a number from 3 to 6,
- $R^1$ denotes hydrogen or methyl, independently for each chain starting from A, and
- X represents one of the radicals

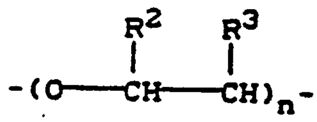

or

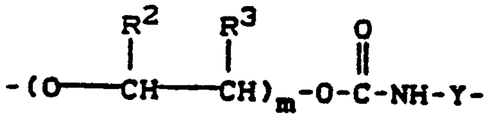

wherein

- n denotes a number from 1 to 5, independently for each chain starting from A,
- m denotes a number from 0 to 5, independently for each chain starting from A,
- $R^2$ and $R^3$ denote hydrogen or hydrogen and methyl, and
- Y is a divalent, straight-chain or branched aliphatic radical which has 2 to 12 carbon atoms and can optionally contain 1 or 2 oxygen bridges,

in dental materials.

2. Use according to Claim 1, characterised in that the (meth)acrylic acid esters are used in tooth-filling compositions.

3. Use according to Claim 1, characterised in that the (meth)acrylic acid esters are used in coatings for teeth.

4. Use according to Claim 1, characterised in that the (meth)acrylic acid esters are used for the preparation of plastic teeth.

5. Use of (meth)acrylic acid esters of the formula (I)

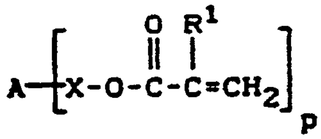

(I)

in which

- A is a straight-chain or branched aliphatic radical having 3 to 20 carbon atoms and optionally containing 1 or 2 oxygen bridges, an araliphatic radical having 7 to 26 carbon atoms or a cycloaliphatic radical having 6 to 26 carbon atoms,
- p represents the number of chains starting from A and denotes a number from 3 to 6,
- $R^1$ denotes hydrogen or methyl, independently for each chain starting from A, and
- X represents one of the radicals

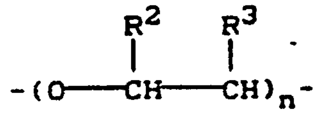

or

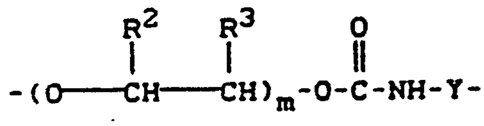

wherein

- n denotes a number from 1 to 5, independently for each chain starting from A,
- m denotes a number from 0 to 5, independently for each chain starting from A,
- $R^2$ and $R^3$ denote hydrogen or hydrogen and methyl, and
- Y denotes a divalent, straight-chain or branched aliphatic radical which has 2 to 12 carbon atoms and can optionally contain 1 or 2 oxygen bridges,

for the preparation of dental materials.

**6.** Dental materials, characterised in that they contain (meth)acrylic acid esters of the formula (I)

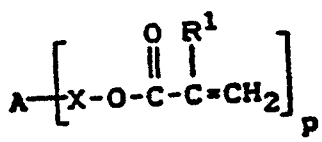

(I)

in which

- A is a straight-chain or branched aliphatic radical having 3 to 20 carbon atoms and optionally containing 1 or 2 oxygen bridges, an araliphatic radical having 7 to 26 carbon atoms or a cycloaliphatic radical having 6 to 26 carbon atoms,
- p represents the number of chains starting from A and denotes a number from 3 to 6,
- $R^1$ denotes hydrogen or methyl, independently for each chain starting from A, and
- X represents one of the radicals

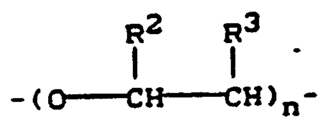

or

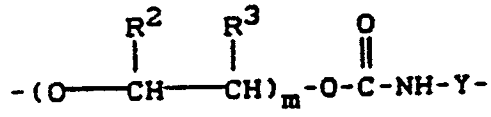

wherein

- n denotes a number from 1 to 5, independently for each chain starting from A,
- m denotes a number from 0 to 5, independently for each chain starting from A,
- $R^2$ and $R^3$ denote hydrogen or hydrogen and methyl, and
- Y denotes a divalent, straight-chain or branched aliphatic radical which has 2 to 12 carbon atoms and can optionally contain 1 or 2 oxygen bridges.

**7.** Dental materials according to Claim 6, characterised in that they contain further comonomers in addition to (meth)acrylic acid eaters of the formula I.

**8.** Dental materials according to Claims 6 and 7, characterised in that they contain (meth)acrylic acid eaters of the formula (I), comonomers and additives known per se and optionally fillers.

**Claims for the following Contracting State : ES**

**1.** Process for the preparation of dental materials, characterised in that (meth)acrylic acid esters of the formula (I)

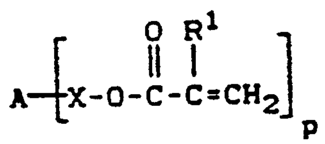

(I)

in which

- A is a straight-chain or branched aliphatic radical having 3 to 20 carbon atoms and optionally containing 1 or 2 oxygen bridges, an araliphatic radical having 7 to 26 carbon atoms or a cycloaliphatic radical having 6 to 26 carbon atoms,
- p represents the number of chains starting from A and denotes a number from 3 to 6,
- $R^1$ denotes hydrogen or methyl, independently for each chain starting from A, and
- X represents one of the radicals

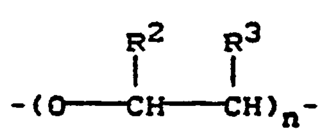

or

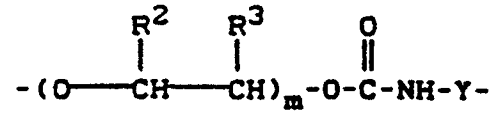

wherein

- n — denotes a number from 1 to 5, independently for each chain starting from A,
- m — denotes a number from 0 to 5, independently for each chain starting from A,
- $R^2$ and $R^3$ — denote hydrogen or hydrogen and methyl, and
- Y — is a divalent, straight-chain or branched aliphatic radical which has 2 to 12 carbon atoms and can optionally contain 1 or 2 oxygen bridges,

are cured in a manner known per se, if desired mixed with comonomers, additives known per se and fillers.

**2.** Process according to Claim 1, characterised in that the dental material is an artificial tooth.

**Revendications**

**Revendications pour les Etats contractants suivants : AT, BE, CH, DE, FR, GB, IT, LI, NL, SE**

**1.** Utilisation d'esters (méth)acryliques de formule (I)

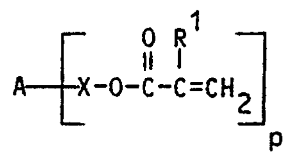

(I),

dans laquelle

A est un reste aliphatique à chaîne droite ou ramifiée en $C_3$-$C_{20}$, contenant éventuellement 1 ou 2 ponts oxygène ou un reste araliphaique en $C_7$-$C_{26}$ ou un reste cycloaliphatique en $C_6$-$C_{26}$,

p est le nombre des chaînes partant de A et représente un nombre de 3 à 6,

$R^1$ représente, indépendamment pour chaque chaîne partant de A, un atome d'hydrogène ou un groupe méthyle et

X représente l'un des restes

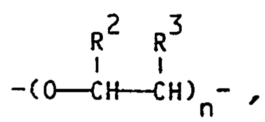

ou

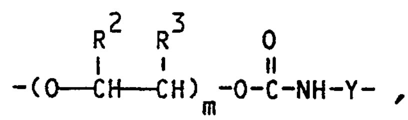

dans lesquels

n représente, indépendamment pour chaque chaîne partant de A, un nombre de 1 à 5,

m représente, indépendamment pour chaque chaîne partant de A, un nombre de 0 à 5,

$R^2$ et $R^3$ représentent l'hydrogène ou l'hydrogène et un groupe méthyle

Y représente un reste aliphatique divalent à chaîne droite ou ramifiée en $C_2$-$C_{12}$, qui peut contenir éventuellement 1 ou 2 ponts oxygène,

dans des matériaux dentaires.

2. Utilisation selon la revendication 1, caractérisée en ce que les esters (méth)acryliques sont utilisés dans des masses de remplissage dentaires.

3. Utilisation selon la revendication 1, caractérisée en ce que les esters (méth)acryliques sont utilisés dans des agents de revêtement pour les dents.

4. Utilisation selon la revendication 1, caractérisée en ce que les esters (méth)acryliques sont utilisés pour la fabrication de dents en matière plastique.

5. Utilisation d'esters (méth)acryliques de formuel (I)

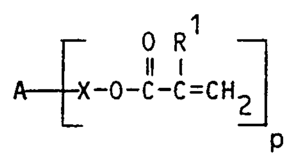

(I)

dans laquelle
A est un reste aliphatique à chaîne droite ou ramifiée en $C_3$-$C_{20}$, contenant éventuellement 1 ou 2 ponts oxygène ou un reste araliphatique en $C_7$-$C_{26}$ ou un reste cycloaliphatique en $C_6$-$C_{26}$,
p est le nombre des chaînes partant de A et représente un nombre de 3 à 6,
$R^1$ représente, indépendamment pour chaque chaîne partant de A, un atome d'hydrogène ou un groupe méthyle et
X représente l'un des restes

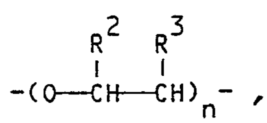

ou

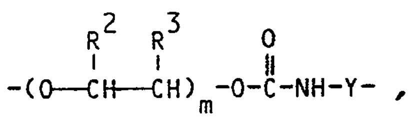

dans lesquels
n représente, indépendamment pour chaque chaîne partant de A, un nombre de 1 à 5,
m représente, indépendamment pour chaque chaîne partant de A, un nombre de 0 à 5,
$R^2$ et $R^3$ représentent l'hydrogène ou l'hydrogène et un groupe méthyle et
Y représente un reste aliphatique divalent à chaîne droite ou ramifiée en $C_2$-$C_{12}$, qui peut contenir éventuellement 1 ou 2 ponts oxygène,
pour la fabrication de matériaux dentaires.

6. Matériaux dentaires, caractérisés en ce qu'ils contiennent des esters (méth)acryliques de formule (I)

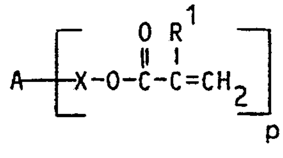

(I),

dans laquelle
A est un reste aliphatique à chaîne droite ou ramifiée en $C_3$-$C_{20}$, contenant éventuellement 1 ou 2 ponts oxygène ou un reste araliphatique en $C_7$-$C_{26}$ ou un reste cycloaliphatique en $C_6$-$C_{26}$,
p est le nombre des chaînes partant de A et représente un nombre de 3 à 6,
$R^1$ représente, indépendamment pour chaque chaîne partant de A, un atome d'hydrogène ou un groupe méthyle et
X représente l'un des restes

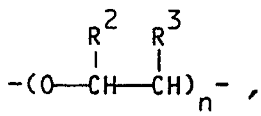

ou

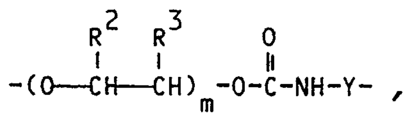

dans lesquels
n représente, indépendamment pour chaque chaîne partant de A, un nombre de 1 à 5,
m représente, indépendamment pour chaque chaîne partant de A, un nombre de 0 à 5,
$R^2$ et $R^3$ représentent l'hydrogène ou l'hydrogène et un groupe méthyle et
Y représente un reste aliphatique divalent à chaîne droite ou ramifiée en $C_2$-$C_{12}$, qui peut contenir éventuellement 1 ou 2 ponts oxygène.

7. Matériaux dentaires selon la revendication 6, caractérisés en ce qu'ils contiennent d'autres comonomères en plus des esters (méth)acryliques.

8. Matériaux dentaires selon les revendications 6 et 7, caractérisés en ce qu'ils contiennent des esters (méth)acryliques de formule (I), des comonomères et des additifs et éventuellement des charges connus en soi.

**Revendications pour l'Etat contractant suivant : ES**

1. Procédé de fabrication de matériaux dentaires, caractérisé en ce que l'on durcit de manière connue des esters (méth)acryliques de formule (I)

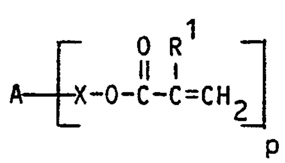 (I) ,

dans laquelle
A est un reste aliphatique à chaîne droite ou ramifiée en $C_3$-$C_{20}$, contenant éventuellement 1 ou 2 ponts oxygène ou un reste araliphatique en $C_7$-$C_{26}$ ou un reste cycloaliphatique en $C_6$-$C_{26}$,
p est le nombre des chaînes partant de A et représente un nombre de 3 à 6,
$R^1$ représente, indépendamment pour chaque chaîne partant de A, un atome d'hydrogène ou un groupe méthyle et
X représente l'un des restes

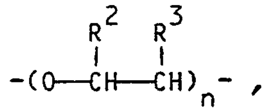

ou

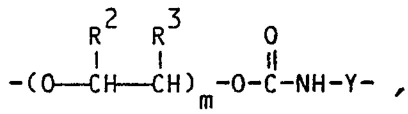

dans lesquels
n représente, indépendamment pour chaque chaîne partant de A, un nombre de 1 à 5,
m représente, indépendamment pour chaque chaîne partant de A, un nombre de 0 à 5,
$R^2$ et $R^3$ représentent l'hydrogène ou l'hydrogène et un groupe méthyle et
Y représente un reste aliphatique divalent à chaîne droite ou ramifiée en $C_2$-$C_{12}$, qui peut contenir éventuellement 1 ou 2 ponts oxygène,
éventuellement en mélange avec des comonomères, des additifs et des charges connus.

**2.** Procédé selon la revendication 1, caractérisé en ce que le matériau dentaire est une dent artificielle.